# EUROPÄISCHE PATENTSCHRIFT

(11) **EP 3 129 167 B1**
(45) Veröffentlichungstag und Bekanntmachung des Hinweises auf die Patenterteilung: **01.09.2021**
(21) Anmeldenummer: 15725227.1
(22) Anmeldetag: 09.04.2015
(51) Int. Cl.: B09C 1/00, G21F 9/00, C12P 5/02, B09C 1/06, F23G 5/027, F23G 5/04, F23G 7/00, G21F 9/18, G21F 9/28

(54) **VERFAHREN ZUR REINIGUNG KONTAMINIERTER UMWELTBRACHEN**
PROCESS FOR CLEANING CONTAMINATED ENVIRONMENTAL FALLOWS
PROCÉDÉ D'ÉPURATION DE FRICHES ENVIRONNEMENTALES CONTAMINÉES

(30) Priorität: 09.04.2014 DE 102014005207
(43) Veröffentlichungstag der Anmeldung: 15.02.2017
(73) Patentinhaber: Niederbacher, Michael, 39031 Bruneck (IT)
(72) Erfinder: Niederbacher, Michael, 39031 Bruneck (IT)
(74) Vertreter: Liebl, Thomas
(86) Internationale Anmeldenummer: PCT/EP2015/000746
(87) Internationale Veröffentlichungsnummer: WO 2015/154874

(56) Entgegenhaltungen:
- WO-A1-99/55803
- DE-A1- 3 921 336
- DE-A1-102006 048 159
- JP-A- 2006 110 540
- JP-A- 2010 104 943
- "CENTRAL EUROPE Project 1CE084P4 ReSOURCE", , 31. Dezember 2010 (2010-12-31), XP055206880, Gefunden im Internet: URL:http://www.central2013.eu/fileadmin/us er_upload/Downloads/outputlib/ReSource_4.2 .5_Biomas_on_contaminated_land_-_study_Fre iberg.pdf [gefunden am 2015-08-07]

## Beschreibung

Die Erfindung bezieht sich auf ein Verfahren zur Reinigung kontaminierter Umweltbrachen, nach Anspruch 1. Störfälle in Kernkraftwerken, wie beispielsweise in Tschernobyl oder in Fukushima, bei denen große Mengen an radioaktivem Material freigesetzt werden, führen zu einer erheblichen Kontamination von Luft, Böden, Wasser und Nahrungsmitteln in der näheren und auch weiteren Umgebung der Kernkraftwerke. Ein derartiger Super-Gau führt dazu, dass insbesondere die landseitige Umgebung derartiger havarierter Kernkraftwerke für Mensch und Tier für eine sehr lange Zeit nicht mehr nutzbar ist und damit eine radioaktiv kontaminierte Umweltbrache vorliegt, die regelmäßig auch als Sperrgebiet bezeichnet wird. Insbesondere die dort auf derartigen kontaminierten Landstrichen bzw. Anbauflächen wachsenden Pflanzen, Sträucher, Bäume etc. (nachfolgend kurz als pflanzliche Biomasse bezeichnet) können keiner weiteren Verwertung, zum Beispiel als Nutz- und/oder Futterpflanze, zugeführt werden, da diese ebenso wie die Böden, auf denen sie wachsen, radioaktiv kontaminiert sind. Der Grund hierfür liegt darin, dass pflanzliche Biomasse radioaktive Stoffe, wie beispielsweise Cäsium, Strontium, Uran und Plutonium, die den Boden radioaktiv kontaminieren, über das Grundwasser aufnehmen, so dass sich die radioaktiven Stoffe auch in der pflanzlichen Biomasse ablagern. Das Gleiche gilt in analoger Weise für Böden, die mit anderen Schadstoffen, zum Beispiel mit Schwermetallen, Dioxinen etc., kontaminiert sind. Auch bei derartigen Umweltbrachen nimmt die pflanzliche Biomasse die Schadstoffe über das Grundwasser auf, so dass sich die Schadstoffe in der pflanzlichen Biomasse ablagern.

Um die Kontaminierung derartiger Umweltbrachen zu reduzieren bzw. zu beseitigen, ist es bereits bekannt, die verseuchten Bodenschichten abzutragen und in einem Endlager zu lagern. Das Problem, das hierbei auftritt, ist, dass für eine derartige Endlagerung der Bodenschichten ein riesiges Volumen benötigt wird, das in der Lage ist, mehrere Millionen Tonnen an verseuchtem Boden aufzunehmen.

Die Studie "CENTRAL EUROPE Project 1CE084P4 ReSOURCE", 31. Dezember 2010(2010-12-31), XP055206880, gefunden im Internet URL: http://www.central2013.eu/fileadmin/user_upload/Downloads/outputlib/ReSour ce_4.2.5_Biomas_on_contaminated_land_-_study_Freiberg.pdf (gefunden am 2015-08-07), befasst sich mit dem Anbau von Biomasse auf verunreinigten Böden und verweist darauf, dass der Einsatz von geernteter Biomasse, die auf verunreinigten Böden angebaut worden ist, in Verbindung mit der Nahrungsmittel- und Futtererzeugung aufgrund gesetzlicher Vorschriften nicht erlaubt ist, dagegen der Anbau von Energiepflanzen juristisch unbedenklich ist. Im weiteren Verlauf der Studie wird darauf verwiesen, unter welche spezielle Biogasanlagen eine sehr hohe Anreicherung des Substrats mit Schwermetallen stattfindet.

Die DE 10 2006 048 159 A1 betrifft eine Vorrichtung und ein Verfahren zur Gewinnung von Energie aus Biomasse, bei dem der ungenützte Energieinhalt des Gärsubstrates eines Fermenters weiter wirtschaftlich geschlossen werden soll. Hierzu sieht die DE 10 2006 048 159 A1 konkret vor, das Substrat aus dem Fermenterbehälter einem Separator zuzuführen, der das Gärsubstrat in eine feste Phase und in eine flüssige Phase aufteilt. Die feste Phase wird dann dem Trockner zugeführt. Nach dem Trocknungsvorgang wird das getrocknete Gärsubstrat einem thermischen Vergaser zugeführt, der aus dem Substrat Koks und Synthesegas produziert. Das Synthesegas wird anschließend, gemischt mit dem Biogas aus dem Fermenter, einem BHKW zur Stromerzeugung zugeführt. An keiner Stelle dieser Druckschrift findet sich ein Hinweis darauf, diese Verfahrensführung zur Reinigung kontaminierter Umweltbrachen einzusetzen. Das Gleiche gilt für die einen ähnlichen Aufbau zeigende JP 2006 110540 A.

Die gattungsbildende DE 39 21 336 A1 betrifft ein Reinigungsverfahren, bei dem der verunreinigte Boden mit schadstoffresistenten, insbesondere schwermetallresistenten Pflanzen bepflanzt wird, die Schadstoffe und/oder radioaktive Stoffe aufnehmen. Gemäß einer ersten Ausführungsform wird vorgeschlagen, aus der Biomasse dieser Pflanzen Methanol zu erzeugen. Falls auf die Herstellung von Methanol verzichtet werden soll, wird die Biomasse getrocknet und unter Energiegewinnung in einer Müllverbrennungsanlage mit einem Abluftfilter verbrannt. Da die in der Asche befindlichen Schwermetalle nicht weiter verwendet werden können, werden sie auf einer Sonderdeponie gelagert.

Demgegenüber ist es Aufgabe der vorliegenden Erfindung, ein Verfahren zur Reinigung kontaminierter Umweltbrachen zur Verfügung zu stellen, mittels dem kontaminierte Biomasse auf kostengünstige und effiziente Weise entsorgt werden kann.

Diese Aufgabe wird gelöst mit den Merkmalen des Anspruchs 1. Vorteilhafte Ausgestaltungen hierzu sind Gegenstand der jeweils darauf rückbezogenen Unteransprüche.

Gemäß Anspruch 1 wird ein Verfahren zur Reinigung kontaminierter Umweltbrachen, vorgeschlagen, bei dem in einer Biogasanlage durch Vergärung von kontaminierter Biomasse, in wenigstens einem Fermenter der Biogasanlage ein kontaminierter, flüssiger und/oder feuchter Gärrest sowie in der Regel nicht kontaminiertes Biogas erzeugt wird. Der kontaminierte, flüssige und/oder feuchte Gärrest wird wenigstens einer Trocknungseinrichtung zugeführt, in der er zu kontaminiertem getrocknetem Gärrest mit einer definiert vorgegebenen Restfeuchtigkeit getrocknet wird. Dieser kontaminierte getrocknete Gärrest wird einer der Biogasanlage zugeordneten Verbrennungs- und/oder Vergasungseinrichtung zugeführt, in der der kontaminierte getrocknete Gärrest verbrannt und/oder vergast wird. Die in der Verbrennungs- und/oder Vergasungseinrichtung anfallende kontaminierte Asche wird abgezogen und separiert und insbesondere einem Endlager zugeführt. Unter einem Endlager ist hier selbstverständlich kein herkömmliches Gärresteendlager einer Biogasanlage zu verstehen, sondern eine spezielle Einrichtung, in der nicht weiter verarbeitbarer bzw. nicht wieder aufbereitbarer Sondermüll, wie zum Beispiel radioaktive Abfälle, deponiert bzw. endgelagert wird.

Besonders bevorzugt ist hierbei eine Verfahrensführung, bei der der kontaminierte getrocknete Gärrest der Verbrennungs- und/oder Vergasungseinrichtung zusammen mit einer definierten Menge eines definierten, zu verbrennenden und/oder zu vergasenden Einsatzstoffes, insbesondere Holz, zugeführt wird. Im Falle von Holz als zu verbrennendem und/oder zu vergasendem Einsatzstoff kann dieses, insbesondere für den Fall, dass es sich hierbei um frisch geschlagenes Holz handelt, in der ohnehin vorgesehenen Trocknungseinrichtung oder in einer separat vorzusehenden Trocknungseinrichtung auf einen definiert vorgegebenen Restfeuchtegehalt getrocknet werden.

Der der Verbrennungs- und/oder Vergasungseinrichtung zusätzlich zum Gärrest wenigstens zeitweise zugeführte, zu verbrennende und/oder zu vergasende Einsatzstoff wird bevorzugt durch kontaminierte und/oder nicht-kontaminierte pflanzliche Biomasse, insbesondere durch kontaminiertes und/oder nicht-kontaminiertes Holz, gebildet. Die pflanzliche Biomasse als zu verbrennender und/oder zu vergasender Einsatzstoff kann gemäß einer besonders bevorzugten Ausführungsvariante auf einer der Biogasanlage und damit der Verbrennungs- und/oder Vergasungseinrichtung zugeordneten kontaminierten (zum Beispiel radioaktiv kontaminierten) und/oder nicht-kontaminierten Anbaufläche angebaut werden. Als pflanzliche Biomasse zum Einsatz in der Verbrennungs- und/oder Vergasungseinrichtung eignen sich neben Hölzern bzw. holzartigen Pflanzen grundsätzlich auch andere nachwachsende Rohstoffe, wie zum Beispiel Miscanthus. Auch kontaminierte und/oder nicht-kontaminierte Alt- und/oder Abrisshölzer können der Verbrennungs- und/oder Vergasungseinrichtung zugeführt werden.

Wie umfangreiche erfinderseitige Versuche gezeigt haben, kann mit der erfindungsgemäßen Verfahrensführung die Kontamination bzw. Strahlungsbelastung von kontaminierten, Böden bzw. Umweltbrachen auf relativ einfache und funktionssichere Weise reduziert werden, wobei gleichzeitig auch die Menge an endzulagerndem Abfall drastisch reduziert werden kann.

Unter einer kontaminierten Umweltbrache im Sinne der vorstehenden Erfindung sollen ausdrücklich nicht nur Festland-Böden verstanden werden, sondern auch kontaminierte Gewässer, in denen zum Beispiel Algen oder andere Wasserpflanzen als pflanzliche Biomasse geerntet werden können.

Die Verbrennungs- und/oder Vergasungseinrichtung kann zum Beispiel eine Verbrennungsanlage sein, in der der kontaminierte getrocknete Gärrest, gegebenenfalls zusammen mit kontaminierten und/oder nicht kontaminiertem Holz, einer Brennkammer zugeführt und dort verbrannt wird. Besonders bevorzugt ist die Verbrennungs- und/oder Vergasungseinrichtung jedoch eine Synthesegaseinrichtung, in der die Einsatzstoffe durch thermische Vergasung mittels eines Vergasungs- und/oder Oxidationsmittels (zum Beispiel Luft, Sauerstoff oder Wasserdampf) in wenigstens einem Reaktor der Synthesegaseinrichtung vergast werden. Das im Rahmen einer derartigen thermischen Vergasung erzeugte Gas bezeichnet man oftmals als "Holzgas", als Synthesegas, als Syngas oder kurz als SNG (die Abkürzung SNG steht für den englischen Begriff: Synthetic Natural Gas). Besonders bevorzugt ist eine Verfahrensführung, bei der der kontaminierte, getrocknete Gärrest der Synthesegaseinrichtung zusammen mit einer definiert vorgegebenen Menge an zu vergasendem Einsatzstoff, zum Beispiel kontaminiertem und/oder nichtkontaminiertem Holz als zu vergasendem Einsatzstoff, zugeführt wird.

Beispielsweise können einer Biogasanlage, zum Beispiel bezogen auf eine Biogasanlage von 1,5 Megawatt, im Jahr ca. 40.000 t (Tonnen) kontaminierter, zum Beispiel radioaktiv kontaminierter, pflanzlicher Biomasse zugeführt werden, während der, vorzugsweise als Synthesegaseinrichtung ausgebildeten, Verbrennungs- und/oder Vergasungseinrichtung ca. 9.000 t pro Jahr an zusätzlichem Holz zugeführt werden, das bevorzugt wenigstens zum Teil ebenfalls kontaminiertes, zum Beispiel radioaktiv kontaminiertes, Wald-, Abfall- und/oder Altholz ist, das bevorzugt auf einer kontaminierten Waldfläche geschlägert oder auf einer kontaminierten Anbaufläche angebaut worden ist, grundsätzlich aber auch jedes andere geeignete, insbesondere auch nicht kontaminierte, Holzmaterial sein kann. Bei einem derartigen Input in die Biogasanlage bzw. in die Verbrennungs- und/oder Vergasungseinrichtung von ca. 50.000 t pro Jahr ergibt sich dann letztendlich lediglich eine kontaminierte Aschemenge von ca. 1.000 bis 1.500 t pro Jahr, die einem Sondermüll-Endlager zugeführt werden muss. Ohne die erfindungsgemäße Verfahrensführung müssten dagegen bei einer Entsorgung der verseuchten Biomasse wenigstens 40.000 t pro Jahr an kontaminierten Abfall endgelagert werden. Wie die zuvor gemachten Ausführungen somit deutlich aufzeigen, kann mit der erfindungsgemäßen Verfahrensführung somit in ganz erheblichem Maße die Menge an endzulagernden Abfällen, insbesondere an endzulagernden radioaktiven Abfällen, reduziert werden. Das Erfordernis von riesigen Endlagern ist daher bei der erfindungsgemäßen Verfahrensführung nicht gegeben, wobei beim Einsatz der erfindungsgemäßen Verfahrensführung in kontaminierten, großflächigen Umweltbrachen zudem eine relativ schnelle Dekontaminierung von großen verseuchten, insbesondere großen radioaktiv verstrahlten, Flächen/Gewässern erfolgen kann, die dann wieder für Mensch und Tier nutzbar gemacht werden können.

An dieser Stelle sei erwähnt, dass die Begrifflichkeit Asche hier in einem weiten Sinne zu verstehen ist und neben der eigentlichen Asche auch sonstige endzulagernde Abfallstoffe enthalten kann, wie zum Beispiel Schlacke etc., um nur ein weiteres Beispiel zu nennen.

Der Verbrennungs- und/oder Vergasungseinrichtung kann optional auch eine Pelletierungseinrichtung vorgeschaltet sein, in der der kontaminierte getrocknete Gärrest und/oder gegebenenfalls der der Verbrennungs- und/oder Vergasungseinrichtung zusammen mit dem Gärrest zugeführte Einsatzstoff pelletiert wird. Dadurch kann dann das zu verbrennende bzw. zu vergasende Material in geeigneter Weise kompaktiert zur Verfügung gestellt werden.

Das in der Biogasanlage und/oder in der als Synthesegaseinrichtung ausgebildeten Verbrennungs- und/oder Vergasungseinrichtung erzeugte Gas wird bevorzugt einer Verwertungs- und/oder Speichereinrichtung zugeführt. Eine derartige Verwertungseinrichtung kann zum Beispiel ein Blockheizkraftwerk (kurz BHKW genannt) sein. Als Speichereinrichtung kann zum Beispiel ein Gasspeicher und/oder ein Gasnetz fungieren.

Grundsätzlich kann gemäß einer besonders bevorzugten erfindungsgemäßen Verfahrensführung vorgesehen sein, dass das von der Verbrennungs- bzw. Vergasungseinrichtung und/oder von der Biogasanlage kommende Gas stromauf und/oder stromab der Verwertungs- und/oder Speichereinrichtung, insbesondere stromauf und/oder stromab eines BHKWs, wenigstens einer Gasreinigungseinrichtung zugeführt wird, in der definiert vorgegebene Gasbestandteile, insbesondere zum Beispiel in dem Gas eventuell vorhandene radioaktive Rückstände entfernt werden können. Eine derartige Gasreinigungseinrichtung ist zum Beispiel ein Aktivkohlefilter in der Zuführleitung zum BHKW und/oder ein Partikelfilter in der vom BHKW wegführenden Abgasleitung. Sowohl die Aktivkohle als auch der Partikelfilter kann dann dem endzulagernden Abfall zugeführt und zusammen mit diesem entsorgt werden.

Besonders bevorzugt ist ein System, bei dem neben den Anlagenkomponenten auch eine kontaminierte Anbaufläche in die Betrachtung und Verfahrensführung mit einbezogen wird. Konkret ist gemäß dieser besonders bevorzugten Ausgestaltung vorgesehen, dass auf einer kontaminierten, zum Beispiel radioaktiv kontaminierten, Anbaufläche pflanzliche Biomasse anbaubar oder angebaut ist, die nach einer definiert vorgegebenen Zeit geerntet wird. Dieser kontaminierten Anbaufläche ist dann die Biogasanlage und damit die Verbrennungs- und/oder Vergasungseinrichtung zumindest versorgungstechnisch und/oder logistisch zugeordnet, und zwar unmittelbar bzw. in unmittelbarer Nähe benachbart zugeordnet oder alternativ aber auch mittelbar, das heißt beabstandet davon, zugeordnet.

Als pflanzliche Biomasse, die auf den kontaminierten Anbauflächen angebaut wird, eignen sich insbesondere schnell wachsende Pflanzen, zum Beispiel Mais und/oder Getreide (zum Beispiel Reis) und/oder Gras und/oder Sonnenblumen. Damit ist sichergestellt, dass die kontaminierten Böden durch diese vorzugsweise schnellwachsenden Pflanzen relativ schnell ausgelaugt werden und somit die Verunreinigungen bzw. die Radioaktivität in diesen kontaminierten Anbauflächen sehr schnell reduziert werden kann.

Gemäß einer besonders bevorzugten erfindungsgemäßen Verfahrensführung wird nach der Ernte der kontaminierten pflanzlichen Biomasse sofort wieder neue pflanzliche Biomasse auf der kontaminierten Anbaufläche angebaut, zum Beispiel auch unter Beachtung einer bestimmten Fruchtfolge, wobei der Anbau- und Erntezyklus so lange wiederholt wird, bis die Kontaminierung bzw. die radioaktive Strahlung der Anbaufläche unter einen definiert vorgegebenen Grenzwert gefallen ist.

Gemäß einer besonders vorteilhaften Verfahrensführung wird vorgeschlagen, dass die von der wenigstens einen Trocknungseinrichtung erzeugte Abluft wenigstens einem Düngemittelreaktor zugeführt wird, in dem der in der Abluft enthaltene Stickstoff, insbesondere Stickstoff in Form von Ammoniak, wenigstens zum Teil mittels eines Reaktionsmittels, insbesondere mittels Schwefelsäure, zu einem Düngemittel, insbesondere zu Ammoniumsulfat, umgesetzt wird. Anstelle der bevorzugten Schwefelsäure kann grundsätzlich auch Phosphorsäure als Reaktionsmittel verwendet werden. In diesem Fall wird dann als Düngemittel Ammoniumphosphat erhalten. Insbesondere in Verbindung mit einer zum Beispiel auf 1,5 Megawatt ausgelegten Biogasanlage kann zum Beispiel eine Düngemittelmenge von ca. 1.500 t pro Jahr erzeugt werden. Grundsätzlich kann das nicht kontaminierte Düngemittel aber auch anderweitig verwendet werden.

Gemäß einer besonders bevorzugten erfindungsgemäßen Verfahrensführung wird das in dem wenigstens einen Düngemittelreaktor erzeugte Düngemittel wieder auf der kontaminierten, insbesondere radioaktiv kontaminierten, Anbaufläche ausgebracht, auf der die der Biogasanlage bzw. der Verbrennungs- und/oder Vergasungseinrichtung zugeführte kontaminierte Biomasse angebaut wird. Bei einer derartigen Verfahrensführung ist auf jeden Fall sichergestellt, dass selbst für den Fall, dass das Düngemittel noch schwach bzw. leicht radioaktiv kontaminiert sein sollte, dieses trotzdem als Düngemittel Verwendung finden kann, um das Wachstum der pflanzlichen Biomasse zu fördern. Der gegebenenfalls durch das Düngemittel erfolgende Eintrag von Schadstoffen bzw. radioaktiver Strahlung ist hierbei so gering, dass dies zu keiner erheblichen bzw. keiner mit der Kontaminierung der Anbaufläche vergleichbaren Bodenkontaminierung kommt, das heißt letztendlich der Einsatz des Düngemittels dazu führt, dass die pflanzliche Biomasse schneller und in einer gewünschten Weise auf der kontaminierten Anbaufläche wachsen kann, um diese mehr und mehr auszulaugen und die Schadstoffbelastung bzw. radioaktive Strahlungsbelastung der Anbaufläche stetig zu verringern.

Als Düngemittelreaktor kann zum Beispiel ein Abluftwäscher (Scrubber) eingesetzt werden.

Ein weiterer besonderer Vorteil der erfindungsgemäßen Verfahrensführung ist, dass die in der Biogasanlage und/oder in der Verbrennungs- und/oder Vergasungseinrichtung anfallende Wärme wenigstens zum Teil der Trocknungseinrichtung zugeführt werden kann und somit bei der Trocknung des kontaminierten, flüssigen bzw. feuchten Gärrestes Verwendung finden kann, wodurch sich der thermische Wirkungsgrad einer erfindungsgemäßen Verfahrensführung wesentlich erhöhen lässt. Beispielsweise kann in Verbindung mit einer auf 1,5 Megawatt ausgelegten Biogasanlage beispielsweise thermische Energie in der Größenordnung von bis zu 15 Mio. kWh pro Jahr erzeugt werden. In Verbindung mit einer auf ca. 1,5 Megawatt ausgelegten Vergasungseinrichtung können sogar bis zu 19 Mio. kWh pro Jahr thermischer Energie erzeugt werden.

Die Erfindung wird nachfolgend anhand einer Zeichnung näher erläutert.

Die einzige Figur zeigt schematisch eine beispielhafte Anlage 1 zur Reduzierung der Strahlungsbelastung von hier beispielhaft radioaktiv kontaminierten Umweltbrachen. Diese Anlage 1 umfasst hier somit eine radioaktiv kontaminierte Anbaufläche 2, auf der pflanzliche Biomasse in Form Hölzern 3 und beispielsweise in Form von Mais, Sonnenblumen, Reis, etc. die nachfolgend kurz Pflanzen 4 genannt werden, angebaut sind und die nach einer definiert vorgegebenen Zeitspannte geerntet werden. Diese Anbaufläche könnte alternativ oder wenigstens zum Teil auch durch ein kontaminiertes, im vorliegenden Beispielfall radioaktiv kontaminiertes, Gewässer gebildet sein, in dem zum Beispiel Algen und/oder andere Wasserpflanzen wachsen. Unmittelbar benachbart zu der radioaktiv kontaminierten Anbaufläche 2, bzw. zumindest systemisch zugeordnet, befindet sich eine lediglich schematisch und beispielhaft dargestellte Biogasanlage 5, die wenigstens einen Fermenter aufweist. Der Biogasanlage 5 wird, wie durch den Pfeil 6 dargestellt, radioaktiv kontaminierte pflanzliche Biomasse in Form der geernteten Pflanzen 4 zugeführt, so dass in der Biogasanlage 5 regelmäßig nicht bzw. nur gering radioaktiv kontaminiertes Biogas 7 sowie radioaktiv kontaminierter, flüssiger bzw. feuchter Gärrest 8 erzeugt wird.

Der radioaktiv kontaminierte Gärrest 8 wird einer Trocknungseinrichtung 9 zugeführt, in der der radioaktiv kontaminierte Gärrest auf eine definiert vorgegebene Restfeuchtigkeit getrocknet wird.

Der so getrocknete, radioaktiv kontaminierte Gärrest 10 wird anschließend optional einer Pelletierungseinrichtung 31 zugeführt, in der er pelletiert wird. Der, gegebenenfalls pelletierte, jedenfalls aber getrocknete, radioaktiv kontaminierte Gärrest 10 wird anschließend einer als Synthesegaseinrichtung ausgebildeten Vergasungseinrichtung 11 zugeführt, in der durch thermische Vergasung mittels eines geeigneten Vergasungs- und/oder Oxidationsmittels, zum Beispiel Luft, in dem wenigstens einem Reaktor der Vergasungseinrichtung 11 Synthesegas 12 erzeugt wird.

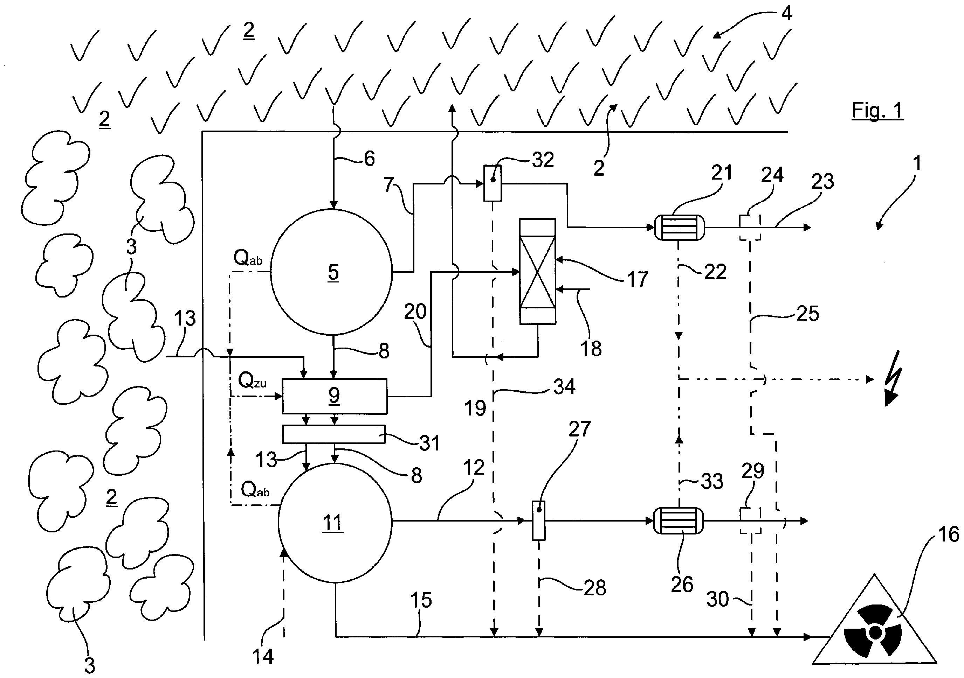

Neben dem getrockneten Gärrest 10 wird der Vergasungseinrichtung 11 hier beispielhaft zudem wenigstens zeitweise auch Holz zugeführt, und zwar zum Beispiel radioaktiv kontaminiertes Abfallholz (Hölzer 3) der Anbaufläche 2 (siehe Pfeil 13 in der Fig. 1). Alternativ oder zusätzlich dazu können der Vergasungseinrichtung 11 aber auch noch jedwede andere geeignete kontaminierte und/oder nicht kontaminierte pflanzliche Biomasse 14, zum Beispiel Hölzer, zugeführt werden, wie dies durch den strichlierten Pfeil in der Fig. 1 lediglich schematisch und beispielhaft dargestellt ist. Diese zugeführten Hölzer können, zum Beispiel im Falle von frischem Schnittholz, vor der Zuführung zur Vergasungseinrichtung 11 auf einen definierten Restfeuchtegehalt getrocknet werden, was in der schematischen Darstellung der Fig. 1 lediglich beispielhaft in Verbindung mit der über die Trocknungseinrichtung 9 geführten Holzzuführung 13 gezeigt ist. Bei trockenen Abfallhölzern, zum Beispiel Abrisshölzern von Holzhäusern, ist dies nicht zwingend erforderlich, wie mit der Holzzuführung 14 lediglich beispielhaft dargestellt ist.

Die in der Vergasungseinrichtung 11 anfallende radioaktiv kontaminierte Asche 15 wird von der Vergasungseinrichtung 11 separiert und abgezogen und einem hier nicht mit einem herkömmlichen Gärresteendlager einer Biogasanlage zu verwechselndem Endlager 16 zur Endlagerung der radioaktiv kontaminierten Asche zugeführt.

Die Abluft 20 aus der Trocknungseinrichtung 9 wird einem als Abluftwäscher ausgebildeten Düngemittelreaktor 17 zugeführt, in dem der in der Abluft enthaltene Stickstoff, insbesondere in Form von Ammoniak, wenigstens zum Teil mittels eines Reaktionsmittels 18, zum Beispiel Schwefelsäure, zu einem Düngemittel 19, zum Beispiel zu Ammoniumsulfat, umgesetzt wird.
Dieses Düngemittel 19 kann dann gemäß einer bevorzugten Ausgestaltung der erfindungsgemäßen Verfahrensführung zur Düngung der radioaktiv kontaminierten Anbaufläche 2 verwendet werden, um das Wachstum der dort neu angebauten Hölzer 3 bzw. Pflanzen 4 zu unterstützen.

Der Abluftwäscher bzw. Düngemittelreaktor 17 ist bevorzugt Bestandteil der Trocknungseinrichtung 9.

Das in der Biogasanlage 5 erzeugte Biogas 7 wird gemäß der beispielhaften Verfahrensführung der Fig. 1 optional einem Gasfilter 32 zugeführt und strömt dann anschließend zu einem BHKW 21, in dem elektrische Energie 22 erzeugt wird. Der Gasfilter 32 ist bevorzugt als Aktivkohlefilter ausgeführt, dessen Aktivkohle dann zum Beispiel im Falle von evt. vorhandenen radioaktiven Verunreinigungen beispielsweise dem Endlager zugeführt werden kann (Pfeil 34).

Die das BHKW 21 verlassenden Abgase 23 können dann zum Beispiel über einen hier lediglich optionalen Partikelfilter 24 geführt werden, um gegebenenfalls noch verbliebende Umweltschadstoffe herauszufiltern bzw. herauszulösen. Eventuell noch vorhandene radioaktive Verunreinigungen können im Rahmen einer Filterentsorgung beispielsweise dem Endlager 16 zugeführt werden (Pfeil 25).

Das von der Vergasungseinrichtung 11 kommende Synthesegas 12 kann ebenfalls stromauf eines BHKWs 26 einem Gasfilter 27, insbesondere einem Aktivkohlefilter, zugeführt werden, in der definierte Gasbestandteile herausgelöst werden können, zum Beispiel eventuell vorhandene radioaktive Reststoffe herausgelöst (Pfeil 28) und letztendlich zusammen mit dem anderen radioaktiven Abfall dem Endlager 16 zugeführt werden können.

Anstelle oder zusätzlich zum Gasfilter 27 stromauf des BHKWs 26 könnte auch wiederum ein Partikelfilter 29 stromab des BHKWs 26 vorgesehen sein, gegebenenfalls auch hier wiederum mit einer Möglichkeit, evtl. vorhandene Reststoffe für die Lagerung in einem Endlager 16 absondern zu können (Pfeil 30).

Die im BHKW 26 erzeugte elektrische Energie 33 wird, ebenso wie die elektrische Energie 22 des BHKWs 21, vorzugsweise einem hier nicht näher dargestellten Stromnetz zugeführt.

Wie dies aus der Fig. 1 weiter schematisch ersichtlich ist, wird die Abwärme (Q_{ab)} aus der Biogasanlage 5 und aus der Vergasungseinrichtung 11 als Zuwärme (Q_{zu}) der Trocknungseinrichtung 9 zugeführt, so dass dadurch der thermische Wirkungsgrad der Anlage 1 insgesamt wesentlich erhöht wird.

Mit der offenbarten Anlage 1 und mit der erfindungsgemäßen Verfahrensführung lassen sich, bezogen auf eine Biogasanlage und eine Vergasungseinrichtung mit jeweils 1,5 Megawatt elektrischer Leistung zum Beispiel folgende Umsatzraten und Leistungen erzielen:

### Input Biogasanlage

| | |
|---|---|
| radioaktiv kontaminierte Maissilage | 10.220 t/a |
| radioaktiv kontaminierte Sonnenblumensilage | 10.220 t/a |
| radioaktiv kontaminierte Grassilage | 10.220 t/a |
| radioaktiv kontaminierte Grünabfälle | 10.220 t/a |

### Input Vergasungseinrichtung

Abfallholz (30% Restfeuchtegehalt an H₂O)8.861 t/a

Dies entspricht einem gesamten Input an Biomasse von 49.741 t/a.

Bei einer derartigen Beschickung der Biogasanlage und der Vergasungseinrichtung erhält man letztendlich nach der Trocknungseinrichtung 4.637 t/a getrockneten, radioaktiv kontaminierten Gärrest.

Nach der Vergasungseinrichtung ergibt sich dann ein jährlicher Ascheabfall von 1.139 t/a, was deutlich zeigt, wie extrem reduziert der letztendliche radioaktive Abfall wird.

Bei einer derartigen Verfahrensführung mit den zuvor genannten Daten kann Ammoniumsulfat als Dünger mit einer Menge von 1.536 t/a erhalten werden.

Zudem kann mittels der Biogasanlage im vorliegenden Beispielfall eine elektrische Energiemenge von 13.237.822 kWh pro Jahr erzielt werden, bei einer Produktion von thermischer Energie in Höhe von 14.346.408 kWh pro Jahr.

Mit der Vergasungseinrichtung können 12.088.800 kWh pro Jahr elektrischer Energie und 18.463.385 kWh pro Jahr thermischer Energie erzeugt werden. Dies ergibt somit eine Gesamtmenge an elektrischer Energie von 25.326.622 kWh pro Jahr und eine Gesamtproduktion von thermischer Energie von 32.809.793 kWh pro Jahr.

Dieses Beispiel belegt die hohe Effektivität der erfindungsgemäßen Verfahrensführung und der offenbarten Anlage auch im Hinblick auf die Produktion von elektrischer und thermischer Energie.

Auch wenn die Erfindung in der Fig. 1 beispielhaft anhand einer radioaktiv kontaminierten Umweltbrache erläutert worden ist, versteht es sich, dass dies nur beispielhaft zu sehen ist und auch jedwede andere Kontaminierung einer Umweltbrache, zum Beispiel mit Schadstoffen wie zum Beispiel Dioxinen, Schwermetallen etc., analog gereinigt werden kann.

### Bezugszeichenliste

| | | | |
|---|---|---|---|
| 1 | Anlage | 25 | Pfeil |
| 2 | Anbaufläche | 26 | BHKW |
| 3 | Hölzern | 27 | Aktivkohlefilter |
| 4 | Pflanzen | 28 | Pfeil |
| 5 | Biogasanlage | 29 | Partikelfilter |
| 6 | Pfeil | 30 | Pfeil |
| 7 | Biogas | 31 | Pelletierungseinrichtung |
| 8 | Gärrest | 32 | Aktivkohlefilter |
| 9 | Trocknungseinrichtung | 33 | elektrische Energie |
| 10 | getrockneter Gärrest | 34 | Pfeil |
| 11 | Vergasungseinrichtung | | |
| 12 | Synthesegas | | |
| 13 | Pfeil | | |
| 14 | pflanzliche Biomasse | | |
| 15 | radioaktive Asche | | |
| 16 | Endlager | | |
| 17 | Düngemittelreaktor | | |
| 18 | Reaktionsmittel | | |
| 19 | Düngemittel | | |
| 20 | Abluft | | |
| 21 | BHKW | | |
| 22 | elektrische Energie | | |
| 23 | Abgase | | |
| 24 | Partikelfilter | | |

## Patentansprüche

1. Verfahren zur Reinigung kontaminierter Umweltbrachen,
mit einer Biogasanlage (5), in der durch Vergärung von kontaminierter Biomasse, in wenigstens einem Fermenter der Biogasanlage (5) ein kontaminierter, flüssiger und/oder feuchter Gärrest (8) sowie Biogas (7) erzeugt wird,
wobei der kontaminierte, flüssige und/oder feuchte Gärrest (8) wenigstens einer Trocknungseinrichtung (9) zugeführt wird, in der er zu kontaminiertem getrocknetem Gärrest (10) mit einer definiert vorgegebenen Restfeuchtigkeit getrocknet wird,
wobei der kontaminierte getrocknete Gärrest (10) einer der Biogasanlage (5) zugeordneten Verbrennungs- und/oder Vergasungseinrichtung (11) zugeführt wird, in der der kontaminierte getrocknete Gärrest (10) verbrannt und/oder vergast wird, und
wobei die in der Verbrennungs- und/oder Vergasungseinrichtung (11) anfallende kontaminierte Asche (15) abgezogen und separiert wird.

2. Verfahren nach Anspruch 1, **dadurch gekennzeichnet, dass** der kontaminierte getrocknete Gärrest (10) der Verbrennungs- und/oder Vergasungseinrichtung (11) zusammen mit einer definiert vorgegebenen Menge an zu verbrennendem und/oder zu vergasendem Einsatzstoff, insbesondere Holz, zugeführt wird, wobei bevorzugt vorgesehen ist, dass im Falle von Holz, insbesondere frisch geschlagenem Holz, als zu verbrennenden und/oder zu vergasenden Einsatzstoff dieses in der oder in einer Trocknungseinrichtung auf einen definiert vorgegebenen Restfeuchtegehalt getrocknet wird.

3. Verfahren nach Anspruch 1 oder 2, **dadurch gekennzeichnet, dass** die Verbrennungs- und/oder Vergasungseinrichtung (11) eine Synthesegaseinrichtung ist, in der durch thermische Vergasung mittels eines Vergasungs- und/oder Oxidationsmittels in wenigstens einem Reaktor der Synthesegaseinrichtung ein Synthesegas (12) erzeugt wird, wobei bevorzugt vorgesehen ist, dass der kontaminierte, getrocknete Gärrest der Synthesegaseinrichtung zusammen mit einer definiert vorgegebenen Menge an zu vergasendem Einsatzstoff, insbesondere Holz, zugeführt wird.

4. Verfahren nach einem der vorhergehenden Ansprüche, **dadurch gekennzeichnet, dass** das in der Biogasanlage (5) und/oder das in einer als Synthesegaseinrichtung ausgebildeten Verbrennungs- und/oder Vergasungseinrichtung (11) erzeugte Gas einer Verwertungs- und/oder Speichereinrichtung, insbesondere wenigstens einem BHKW (21, 26) als Verwertungseinrichtung und/oder einem Gasspeicher als Speichereinrichtung und/oder einem Gasnetz als Speichereinrichtung, zugeführt wird.

5. Verfahren nach Anspruch 4, **dadurch gekennzeichnet, dass** das von der Verbrennungs- und/oder Vergasungseinrichtung (11) und/oder das von der Biogasanlage (5) kommende Gas stromauf und/oder stromab der Verwertungs- und/oder Speichereinrichtung, insbesondere stromauf und/oder stromab eines BHKWs, wenigstens einer Gasreinigungseinrichtung (24, 27, 29) zugeführt wird, in der definiert vorgegebene Gasbestandteile, insbesondere in dem Gas eventuell vorhandene radioaktive Rückstände, entfernt werden, wobei bevorzugt vorgesehen ist, dass wenigstens ein Aktivkohlefilter in der Zuführleitung zum BHKW (21, 26) und/oder wenigstens ein Partikelfilter in der vom BHKW (21, 26) wegführenden Abgasleitung angeordnet ist.

6. Verfahren nach einem der vorhergehenden Ansprüche, **dadurch gekennzeich net,**
dass eine kontaminierte, insbesondere radioaktiv kontaminierte, Anbaufläche (2) vorgesehen ist, auf der pflanzliche Biomasse anbaubar ist oder angebaut ist und nach einer definiert vorgegebenen Zeit geerntet wird,
dass die Biogasanlage (5) der kontaminierten Anbaufläche (2) unmittelbar oder mittelbar zugeordnet ist, wobei in der Biogasanlage durch Vergärung der geernteten kontaminierten pflanzlichen Biomasse in dem wenigstens einem Fermenter der Biogasanlage (5) der kontaminierte, flüssige und/oder feuchte Gärrest (8) sowie Biogas (7) erzeugt wird.

7. Verfahren nach einem der Ansprüche 2 bis 6, **dadurch gekennzeichnet, dass** der der Verbrennungs- und/oder Vergasungseinrichtung (11) zusätzlich zum Gärrest wenigstens zeitweise zugeführte, zu verbrennende und/oder zu vergasende Einsatzstoff durch kontaminierte und/oder nicht-kontaminierte pflanzliche Biomasse, insbesondere durch kontaminiertes und/oder nicht-kontaminiertes Holz, gebildet ist, die auf einer der Biogasanlage (5) und damit der Verbrennungs- und/oder Vergasungseinrichtung (11) zugeordneten kontaminierten und/oder nicht-kontaminierten Anbaufläche (2) angebaut worden ist.

8. Verfahren nach einem der vorhergehenden Ansprüche, **dadurch gekennzeichnet, dass** der Biogasanlage (5) kontaminierter Mais und/oder kontaminiertes Getreide und/oder kontaminiertes Gras und/oder kontaminierte Sonnenblumen und/oder kontaminierte Grünabfälle als kontaminierte, insbesondere als radioaktiv kontaminierte, pflanzliche Biomasse zugeführt wird, wobei bevorzugt vorgesehen ist, dass die kontaminierte pflanzliche Biomasse auf einer der Biogasanlage (5) zugeordneten kontaminierten, insbesondere radioaktiv kontaminierten, Anbaufläche (2) angebaut und geerntet worden ist.

9. Verfahren nach einem der Ansprüche 6 bis 8, **dadurch gekennzeich** net,
dass nach der Ernte der pflanzlichen Biomasse wieder neue pflanzliche Biomasse auf der kontaminierten Anbaufläche (2) angebaut wird, und
dass der gesamte Anbau- und Erntezyklus solange wiederholt wird, bis die Kontamination, insbesondere die radioaktive Strahlung, der Anbaufläche (2) unter einen definiert vorgegebenen Grenzwert gefallen ist.

10. Verfahren nach einem der vorhergehenden Ansprüche, **dadurch gekennzeichnet, dass** die von der wenigstens einen Trocknungseinrichtung (9) erzeugte Abluft wenigstens einem Düngemittelreaktor (17) zugeführt wird, in dem der in der Abluft enthaltene Stickstoff, insbesondere Stickstoff in Form von Ammoniak, wenigstens zum Teil mittels eines Reaktionsmittels, insbesondere mittels Schwefelsäure, zu einem Düngemittel, insbesondere zu Ammoniumsulfat, umgesetzt wird.

11. Verfahren nach Anspruch 10, **dadurch gekennzeichnet, dass** das in dem wenigstens einen Düngemittelreaktor (17) erzeugte Düngemittel auf einer Anbaufläche (2) ausgebracht wird, auf der die der Biogasanlage (5) und/oder der Verbrennungs- und/oder Vergasungseinrichtung (11) zugeführte kontaminierte, insbesondere radioaktiv kontaminierte, Biomasse angebaut wird.

12. Verfahren nach einem der vorhergehenden Ansprüche, **dadurch gekennzeichnet, dass** die in der Biogasanlage (5) und/oder in der Verbrennungs- und/oder Vergasungseinrichtung (11) anfallende Wärme wenigstens zum Teil der Trocknungseinrichtung (9) zugeführt wird.

## Claims

1. Method for the cleaning of contaminated environmental fallows,
with a biogas installation (5), in which a contaminated, liquid and/or moist fermentation residue (8) as well as biogas (7) is produced by fermentation of contaminated biomass in at least one fermenter of the biogas installation (5),
wherein the contaminated, liquid and/or moist fermentation residue (8) is fed to at least one drying device (9), in which it is dried to contaminated dried fermentation residue (10) with a defined predetermined residual moisture content,
wherein the contaminated dried fermentation residue (10) is fed to an incineration and/or gasification device (11) associated with the biogas installation (5), in which the contaminated dried fermentation residue (10) is incinerated and/or gasified, and
wherein the contaminated ash (15) occurring in the incineration and/or gasification device (11) is drawn off and separated.

2. Method according to claim 1, **characterized in that** the contaminated dried fermentation residue (10) is fed to the incineration and/or gasification device (11) together with a defined predetermined quantity of feed material to be incinerated and/or gasified, in particular wood, it being preferably provided that, in the case of wood, in particular freshly cut wood, as feed material to be incinerated and/or gasified, said wood is dried in or in a drying device to a defined predetermined residual moisture content.

3. Method according to claim 1 or 2, **characterized in that** the incineration and/or gasification device (11) is a synthesis gas device in which a synthesis gas (12) is produced by thermal gasification by means of a gasification and/or oxidation agent in at least one reactor of the synthesis gas device, it preferably being provided that the contaminated, dried fermentation residue is supplied to the synthesis gas device together with a defined, predetermined quantity of a feed material to be gasified, in particular wood.

4. Method according to any one of the preceding claims, **characterized in that** the gas produced in the biogas plant (5) and/or the gas produced in an incineration and/or gasification device (11) configured as a synthesis gas device is fed to a utilisation and/or storage device, in particular to at least one CHP unit (21, 26) as utilisation device and/or to a gas storage facility as storage device and/or to a gas grid as storage device.

5. Method according to claim 4, **characterized in that** the gas coming from the incineration and/or gasification device (11) and/or the gas coming from the biogas plant (5) is fed upstream and/or downstream of the utilisation and/or storage device, in particular upstream and/or downstream of a CHP unit, to at least one gas purification device (24, 27, 29), in which defined predetermined gas constituents, in particular radioactive residues which may be present in the gas, are removed, it being preferably provided that at least one active carbon filter is arranged in the feed line to the CHP unit (21, 26) and/or at least one particle filter is arranged in the offgas line leading away from the CHP unit (21, 26).

6. Method according to any one of the preceding claims, **characterized**
**in that** a contaminated, in particular radioactively contaminated, cultivation area (2) is provided on which vegetable biomass is growable or cultivated and is harvested after a defined predetermined time,
**in that** the biogas installation (5) is directly or indirectly associated with the contaminated cultivation area (2), with the contaminated, liquid and/or moist fermentation residue (8) and biogas (7) being produced in the biogas installation by fermentation of the harvested contaminated vegetable biomass in the at least one fermenter of the biogas installation (5).

7. Method according to any one of the claims 2 to 6, **characterized in that** the feeding material to be incinerated and/or gasified, which is fed at least temporarily to the incineration and/or gasification device (11) in addition to the fermentation residue, is formed by contaminated and/or non-contaminated vegetable biomass, in particular by contaminated and/or non-contaminated wood, which has been cultivated on a contaminated and/or non-contaminated cultivation area (2) associated with the biogas installation (5) and thus with the incineration and/or gasification device (11).

8. Method according to any one of the preceding claims, **characterized in that** contaminated corn and/or contaminated grain and/or contaminated grass and/or contaminated sunflowers and/or contaminated green waste is fed to the biogas installation (5) as contaminated, in particular as radioactively contaminated, vegetable biomass, it being preferably provided that the contaminated vegetable biomass has been cultivated and harvested on a contaminated, in particular radioactively contaminated, cultivation area (2) associated with the biogas installation (5).

9. Method according to any one of claims 6 to 8, **characterized in,**
**that** after harvesting the vegetable biomass, new vegetable biomass is again cultivated on the contaminated cultivation area (2), and
in that the entire cultivation and harvesting cycle is repeated until the contamination, in particular the radioactive radiation, of the cultivation area (2) has fallen below a defined predetermined threshold value.

10. Method according to any one of the preceding claims, **characterized in that** the waste air produced by the at least one drying device (9) is fed to at least one fertiliser reactor (17), in which the nitrogen contained in the waste air, in particular nitrogen in the form of ammonia, is converted at least in part by means of a reaction agent, in particular by means of sulphuric acid, to a fertiliser, in particular to ammonium sulphate.

11. Method according to claim 10, **characterized in that** the fertiliser produced in the at least one fertiliser reactor (17) is spread on a cultivation area (2) on which the contaminated, in particular radioactively contaminated, biomass supplied to the biogas installation (5) and/or the incineration and/or gasification device (11) is cultivated.

12. Method according to any one of the preceding claims, **characterized in that** the heat accumulating in the biogas installation (5) and/or in the incineration and/or gasification installation (11) is at least partly supplied to the drying device (9).

## Revendications

1. Procédé d'épuration de friches environnementales contaminées,
comprenant une installation de production de biogaz (5), dans laquelle un résidu de fermentation liquide et/ou humide contaminé (8) ainsi que du biogaz (7) sont produits par fermentation de biomasse contaminée, dans au moins un fermenteur de l'installation de production de biogaz (5),
le résidu de fermentation liquide et/ou humide contaminé (8) étant introduit dans au moins un appareil de séchage (9), dans lequel il est séché en un résidu de fermentation contaminé séché (10) ayant une humidité résiduelle prédéterminée de manière définie,
le résidu de fermentation contaminé séché (10) étant introduit dans un appareil de combustion et/ou de gazéification (11) associé à l'installation de production de biogaz (5), dans lequel le résidu de fermentation contaminé séché (10) est brûlé et/ou gazéifié, et
les cendres contaminées (15) formées dans l'appareil de combustion et/ou de gazéification (11) étant extraites et séparées.

2. Procédé selon la revendication 1, **caractérisé en ce que** le résidu de fermentation contaminé séché (10) est introduit dans l'appareil de combustion et/ou de gazéification (11) conjointement avec une quantité prédéterminée de manière définie de matière première à brûler et/ou à gazéifier, notamment de bois, il étant de préférence prévu que dans le cas de bois, notamment de bois fraîchement abattu, en tant que matière première à brûler et/ou à gazéifier, celui-ci soit séché dans l'appareil de séchage ou dans un appareil de séchage à une teneur en humidité résiduelle prédéterminée de manière définie.

3. Procédé selon la revendication 1 ou 2, **caractérisé en ce que** l'appareil de combustion et/ou de gazéification (11) est un appareil de production de gaz de synthèse, dans lequel un gaz de synthèse (12) est produit par gazéification thermique au moyen d'un agent de gazéification et/ou d'oxydation dans au moins un réacteur de l'appareil de production de gaz de synthèse, il étant de préférence prévu que le résidu de fermentation contaminé séché soit introduit dans l'appareil de production de gaz de synthèse conjointement avec une quantité prédéterminée de manière définie de matière première à gazéifier, notamment de bois.

4. Procédé selon l'une quelconque des revendications précédentes, **caractérisé en ce que** le gaz produit dans l'installation de production de biogaz (5) et/ou dans un appareil de combustion et/ou de gazéification (11) configuré en tant qu'appareil de production de gaz de synthèse est introduit dans un appareil de transformation et/ou de stockage, notamment au moins une centrale de production combinée de chaleur et d'électricité (21, 26) en tant qu'appareil de transformation et/ou un réservoir de gaz en tant qu'appareil de stockage et/ou un réseau de gaz en tant qu'appareil de stockage.

5. Procédé selon la revendication 4, **caractérisé en ce que** le gaz provenant de l'appareil de combustion et/ou de gazéification (11) et/ou de l'installation de production de biogaz (5) est introduit, en amont et/ou en aval de l'appareil de transformation et/ou de stockage, notamment en amont et/ou en aval d'une centrale de production combinée de chaleur et d'électricité, dans au moins un appareil d'épuration de gaz (24, 27, 29), dans lequel des constituants gazeux prédéterminés de manière définie, notamment des résidus radioactifs éventuellement présents dans le gaz, sont éliminés, il étant de préférence prévu qu'au moins un filtre à charbon actif soit agencé dans la conduite d'alimentation de la centrale de production combinée de chaleur et d'électricité (21, 26) et/ou qu'au moins un filtre à particules soit agencé dans la conduite de gaz d'échappement sortant de la centrale de production combinée de chaleur et d'électricité (21, 26).

6. Procédé selon l'une quelconque des revendications précédentes, **caractérisé en ce que**
une surface de culture contaminée (2), notamment contaminée radioactivement, est prévue, sur laquelle une biomasse végétale peut être cultivée ou est cultivée et est récoltée après un temps prédéterminé de manière définie,
**en ce que** l'installation de production de biogaz (5) est associée directement ou indirectement à la surface de culture contaminée (2), le résidu de fermentation liquide et/ou humide contaminé (8), ainsi que du biogaz (7) étant produits dans l'installation de production de biogaz par fermentation de la biomasse végétale contaminée récoltée dans l'au moins un fermenteur de l'installation de production de biogaz (5).

7. Procédé selon l'une quelconque des revendications 2 à 6, **caractérisé en ce que** la matière première à brûler et/ou à gazéifier introduite au moins périodiquement en plus du résidu de fermentation dans l'appareil de combustion et/ou de gazéification (11) est formée par une biomasse végétale contaminée et/ou non contaminée, notamment par du bois contaminé et/ou non contaminé, qui a été cultivée sur une surface de culture contaminée et/ou non contaminée (2) associée à l'installation de production de biogaz (5) et par conséquent à l'appareil de combustion et/ou de gazéification (11).

8. Procédé selon l'une quelconque des revendications précédentes, **caractérisé en ce que** du maïs contaminé et/ou du blé contaminé et/ou de l'herbe contaminée et/ou des tournesols contaminés et/ou des déchets verts contaminés sont introduits dans l'installation de production de biogaz (5) en tant que biomasse végétale contaminée, notamment contaminée radioactivement, il étant de préférence prévu que la biomasse végétale contaminée ait été cultivée et récoltée sur une surface de culture contaminée (2), notamment contaminée radioactivement, associée à l'installation de production de biogaz (5).

9. Procédé selon l'une quelconque des revendications 6 à 8, **caractérisé en ce que**
après la récolte de la biomasse végétale, une nouvelle biomasse végétale est de nouveau cultivée sur la surface de culture contaminée (2), et
**en ce que** l'ensemble du cycle de culture et de récolte est répété jusqu'à ce que la contamination, notamment le rayonnement radioactif, de la surface de culture (2) ait chuté en dessous d'une valeur seuil prédéterminée de manière définie.

10. Procédé selon l'une quelconque des revendications précédentes, **caractérisé en ce que** l'air sortant produit par l'au moins un appareil de séchage (9) est introduit dans au moins un réacteur à engrais (17), dans lequel l'azote contenu dans l'air sortant, notamment l'azote sous la forme d'ammoniac, est au moins partiellement converti au moyen d'un agent de réaction, notamment au moyen d'acide sulfurique, en un engrais, notamment en sulfate d'ammonium.

11. Procédé selon la revendication 10, **caractérisé en ce que** l'engrais produit dans l'au moins un réacteur à engrais (17) est épandu sur une surface de culture (2), sur laquelle la biomasse contaminée, notamment contaminée radioactivement, introduite dans l'installation de production de biogaz (5) et/ou dans l'appareil de combustion et/ou de gazéification (11), est cultivée.

12. Procédé selon l'une quelconque des revendications précédentes, **caractérisé en ce que** la chaleur formée dans l'installation de production de biogaz (5) et/ou dans l'appareil de combustion et/ou de gazéification (11) est au moins partiellement introduite dans l'appareil de séchage (9).
